# EUROPEAN PATENT APPLICATION

(11) **EP 1 634 656 A1**
(43) Date of publication of application: **15.03.2006**
(21) Application number: 04077534.8
(22) Date of filing: 13.09.2004
(51) Int. Cl.: B08B 9/043, A61B 1/12

(54) **Ultrasound lumen cleaning technique**

(71) Applicant: Nederlandse Organisatie voor Toegepast-Natuurwetenschappelijk Onderzoek TNO, 2628 VK Delft (NL)
(72) Inventor: Wieringa, Fokko Pieter, 6921 JA Duiven (NL); Bezemer, Robert Alfred, 2401 GL Alphen aan den Rijn (NL)
(74) Representative: Winckels, Johannes Hubertus F.

(57) **Abstract**

The interior surface of a long and narrow space such as the lumen (16) of an endoscope or catheter is cleaned by moving a liquid-filled, ultrasound-resonant cavity (14) through the interior space. An energy supply channel, such as an optical fibre (12), is connected between the cavity (14) and the outside of the object. Excitation energy is fed through the energy supply channel, with a power level that is modulated with a frequency that substantially corresponds to an ultrasound-resonance frequency of the cavity (14). The excitation energy is converted in the cavity (14) to pressure variations in the liquid in the cavity (14), so that an ultrasound standing wave is excited in the cavity (14). Ultrasound pressure variations are fed from the cavity (14) to the interior surface.

## Description

The invention relates to a cleaning technique for cleaning the lumen of a long and narrow space, in particular of medical equipment with such a space, such as for example an endoscope. However, the invention can also be applied to cleaning of other types of equipment where cleanliness of parts is crucial, such as ultra high vacuum systems.

German patent application DE 4432683 describes a technique for cleaning the interior surface of an endoscope or catheter using ultrasonic energy. An ultrasound waveguide is provided to guide the ultrasound energy from outside the endoscope or catheter to its interior.

The use of ultrasound for cleaning medical equipment has long been known. Normally, the medical equipment is submerged in a bath of water and ultrasound waves are excited in the water to remove material such as blood, tissue and other biological material from the surface of the equipment. However, it is difficult to apply such techniques to cleaning of interior surfaces in narrow interior spaces, because it is difficult to deliver sufficient ultrasound power in the interior spaces. This problem is aggravated when the interior of a flexible and/or bent tube has to be cleaned, because it is difficult to reach the entire interior surfaces of such a tube. E.g. flexible endoscopes contain air-filled compartments, which dampen externally applied ultrasound.

The use of an ultrasonic waveguide to supply ultrasound power to the interior, as described in DE 4432683, increases the amount of ultrasound pressure that can be generated on the interior spaces. Nevertheless the amount of power that can be delivered in this way is limited. According to DE 4432683 the amount of power surprisingly was sufficient to remove some material. However, the power limits the extent to which material can be removed. Furthermore, ultrasonic waveguides mechanically vibrate as a whole and can cause serious wear of the surfaces to be cleaned if mechanical contact occurs. Especially for flexible endoscopes this is a drawback of the ultrasonic waveguide.

DE 4432683 describes how more material can be removed using a synergetic effect between cleaning chemicals and the ultrasound energy. The cleaning chemicals are provided in the form of photosensitive material in the liquid that is supplied to the interior of the catheter or endoscope. A laser source is used to activate the photosensitive material, forming radicals that destroy biological material on the interior surface of the endoscope or catheter. According to DE 4432683 the ultrasound energy prolongs the lifetime of the radicals, thus increasing their effect. In DE 4432683 the laser source is not used to induce or significantly increase the pressure amplitude of the ultrasound waves.

Among others it is an object of the invention to improve cleaning of the interior surfaces of tube shaped equipment with sound waves, by increasing the amplitude of the sound pressure that can locally be applied to the interior surfaces.

Among others it is an object of the invention to improve cleaning of the interior surfaces of flexible tubes.

A method and apparatus according to the invention are set forth in the independent claims. According to the invention, a sound-resonant cavity is moved through the interior of the tube such as the lumen of an endoscope. The cavity is attached to an energy supply channel, through which modulated energy is supplied that is converted to excite a standing sound wave in the cavity. Preferably, ultrasound waves are excited, which, as used herein, are sound waves ate higher frequency than audible sound, that are effective for cleaning. Preferably, the energy supply channel is an optical fibre, through which amplitude modulated light is supplied to excite the standing sound wave, the light modulation frequency corresponding substantially to a sound resonance frequency of the cavity. Preferably, the output surface of the optical fibre is concave, so that there is a natural focus of the sound waves. Preferably the modulated light is converted to pressure variations by directly heating the liquid in the cavity through absorption of the light. Alternatively, light-absorbing material may be provided in contact with liquid in the cavity. Optically induced liquid to vapour transformation can create shorter pressure rise times, leading to more higher harmonics, than for example piezo electric excitation of sound waves.

During cleaning the assembly of the cavity and the energy supply channel is moved through the interior space of the tube, for example by pulling on the cavity with the energy supply channel. Energy is supplied from outside the tube through the energy supply channel and converted to pressure variations in the cavity at the different positions that the cavity passes along when it is moved. Pressure variations due to the standing wave are coupled to the outside of the cavity, for example via a hole in the wall of the cavity, or via a valve that opens when the pressure exceeds a threshold value. From the cavity the pressure variations reach the inner surface of the tube, so that material is ultrasonically detached from the inner surface. The material is then washed out by a flow of liquid through the tube. Preferably, the energy supply channel is combined with a supply or drainage tube to supply or drain liquid close by where the pressure variations leave the cavity. In this way the supply or drainage tube can be moved along with the cavity through the inner space that is cleaned.

Preferably, a UV light supply channel is joined to the energy supply channel so that it passes UV light to a location where ultrasound pressure variations from the cavity are applied to the inner surface of the tube. In this way the inner surface can be disinfected with UV light immediately after material has been detached. The UV light supply channel is provided so that it can be moved along with the cavity through the inner space that is cleaned. An additional optical fibre may be provided for this purpose for example, or the UV light may be fed through the optical fibre that is used to excite the ultrasound standing wave in the cavity.

In an embodiment the cleaning apparatus comprises a bundle of optical fibres, each coupled to a respective cavity to excite standing ultrasound waves in the cavities by means of modulated light. In this way only a small amount of ultrasound energy needs to be leaked from each of the cavities to expose a relatively large inner surface of the tube. As a result a high ultrasound pressure amplitude can be reached.

In another embodiment the cleaning apparatus comprises multiple UV light supply channels, each passing UV light to a location where ultrasound pressure variations from the cavity are applied to the inner surface of the tube.

These and other objects and advantageous aspects of the invention will be illustrated using non-limited examples that are described by reference to the following figures
- Figure 1: shows a cleaning apparatus
- Figure 2: shows a tip of an embodiment of the cleaning apparatus
- Figure 2a: shows an alternative tip
- Figure 2b: shows a tip of an embodiment of the cleaning apparatus, with two energy supply channels connected to a single cavity
- Figure 3: shows another tip
- Figure 4: shows a cross-section of part of a cleaning apparatus, with multiple energy supply channels and multiple cavities
- Figure 5: shows a tip of an embodiment of the cleaning apparatus, with an adjacent UV light supply channel
- Figure 5a: shows a cross-section of part of a cleaning apparatus, with multiple UV light supply channels
- Figure 6: shows a tip of an embodiment of the cleaning apparatus, with a light-absorbing layer at the fibre tip.

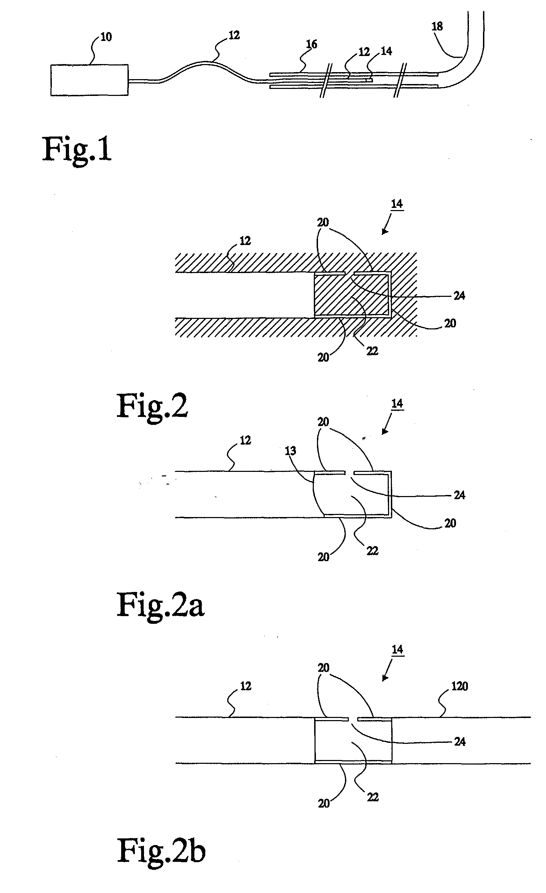

Figure 1 shows a cleaning apparatus for cleaning the inside of a tube 16 that may be used as part of an endoscope, a catheter or other medical equipment.

The cleaning apparatus comprises a laser source 10, a flexible optical fibre 12 and a liquid filled cavity 14. A supply 18 of liquid is coupled to tube 16. Optical fibre 12 is coupled to laser source 10 and cavity 14 is provided at the tip of optical fibre 12.

Figure 2 shows the tip of optical fibre 12 and cavity 14 in more detail. Cavity 14 comprises a space 22 that is enclosed on one side by the surface at the tip of optical fibre 12 and on the other sides by walls 20 that are attached to optical fibre 12. The space 22 between the tip of optical fibre 12 and walls 20 is filled with liquid. In a sidewall 20 of the cavity a hole 24 is provided.

Although tube 16 of the endoscope, catheter or other medical equipment is shown as a straight tube, it should be understood that this tube is typically flexible, and that it may exhibit any number of bends. Typically, tube 16 has an inside diameter between a few millimetres (e.g. more than one millimetre) and a centimetre. The length of tube 16 is typically considerably greater than its diameter, e.g. between ten centimetre and more than a metre. In one example the length of cavity 14 is 15 millimetres, however other lengths may be used (e.g. 1.5 millimetre). The cavity walls may be made of metal or plastic for example, the interior space being formed by removal of material from a solid tube part, or by casting for example.

In operation, after tube 16 has been used in contact with a patient, or has otherwise been contaminated, tube 16 is attached to a supply 18 of a liquid such as water, so that the liquid flows through tube 16. Cavity 14 is filled with a similar liquid. Fibre 12 and cavity 14 are drawn through tube 16, or pushed through tube 16, while laser source 10 transmits amplitude-modulated light through fibre 12.

The wavelength of the laser source is selected in an absorption band of the liquid in cavity 14. A wavelength of 2940 nm is used for example (e.g. an Er:YaG laser) in combination with water in cavity 14. The modulation frequency of the light is selected substantially equal to an acoustic resonance frequency of cavity 14 when filled with the liquid (e.g. within a range wherein the amplitude response in the cavity is more than half the maximum response at the resonance frequency). The resonance frequency depends on the dimensions of cavity 14. Preferably the dimensions of the cavity 14 are selected so that the cavity is resonant at a frequency that is known to have ultrasonic cleaning effect, e.g. between 40 and 100 kHz. In one example the resonance frequency is 50 kHz for a cavity 14 with a length of 15 millimetres. However, higher frequencies and correspondingly smaller cavities may be used (e.g. 1.5 MHz with a cavity length of 0.5 millimetre). All frequencies above 20 kHz will be referred to as "ultrasonic" herein. The resonance frequency that must be used for a given cavity 10 can easily be determined experimentally.

Preferably the modulated light is pulsed light, with light pulses at a frequency substantially equal to the ultrasound resonance frequency of cavity 14. It will be appreciated that cavity 14 typically has a plurality of resonance frequencies that may each be used, for example odd multiples of a ground resonance frequency. In an alternative embodiment a corresponding modulation frequency may be used that is a substantially a sub-harmonic of the resonance frequency (e.g. one half, one third or one fifth), when the pulses induce sufficient harmonics in the cavity to excite the excitation.

The laser light enters cavity 14 from optical fibre 12, where absorption of the light by the liquid causes rapid heating of the liquid in cavity 14. Preferably a laser power level is used in the pulses that is sufficiently high to heat the liquid quickly to above its boiling point, so that vapour bubbles appear as a result of heating. Typically absorption and bubbles will occur mainly in a region in cavity 14 adjacent to the interface with the fibre, when the wavelength is selected in an absorption band of the liquid. The bubbles excite pressure waves that result in a wave pattern of ultrasound standing waves in cavity 14. Because light pulses are applied at the resonance frequency of the cavity, the amplitude of the standing waves rises to a considerable size.

Hole 24 is located at or near a location of maximum amplitude of the standing waves in cavity 14, i.e. substantially midway between the two ends of the cavity if a first order mode with one maximum is excited. Location near the location of maximum amplitude has the effect that sound waves are excited in the liquid adjacent to cavity 14. These sound waves propagate to the inner surface of the tube 16, where they serve to detach material from the inner surface. The material is washed out by liquid current through the tube caused by the supply 18 of liquid. Preferably, cleaning and/or disinfecting chemicals are added to the supplied liquid to improve cleaning.

Cavity 14 may be realized for example by gluing a hollow part with walls 20 to the tip surface of optical fibre 12, or by shoving such a part with the walls partly over the circumference of optical fibre 12, optionally also gluing or shrinking the walls to the circumference. Alternatively, walls 20 may comprise cladding material of optical fibre 12, from which a central part of the optical fibre 12 has been pulled back, or removed by chemical or physical processing.

Preferably, a plurality of holes 24 is provided in walls 20 around the perimeter of cavity 14, so that sound waves are emitted in a plurality of directions transverse to the axis of optical fibre 12 and cavity 14. Preferably a sufficient number of holes are provided to emit sound waves substantially in all directions circling the axis. In this way the inner surface of tube 16 can be cleaned without rotating cavity 14 around the axis.

Figure 2a shows another embodiment of the tip of optical fibre 12 and cavity 14. In this embodiment the output surface of the fibre (the surface that forms the interface with the liquid in cavity 14) is concave, shaped to focus ultrasound waves from the surface of the fibre to a point near hole 24. In operation the focussing results in a resonant mode shape with a local maximum near the holes. The shape of the output surface can be chosen such that the local maximum is off the length axis of the fibre, displaced from the axis towards the hole 24. The surface may be of parabolic shape for example, with an off fibre-axis focus near hole 24. In this way the amplitude of the sound waves that emerge from the hole 24 is increased. Instead of the concave shape of the output surface of the fibre 12, or in addition to the concave output surface of the fibre 12, the wall 20 of cavity opposite the output surface may have a concave shape to realize a similar focussing effect.

When more than one hole is provided in the walls 20 of cavity 14, preferably the output surface of fibre 12 and/or the opposite wall 20 has a plurality of concave parts, each shaped to create a local maximum in the mode shape near a respective one of the holes 24.

Figure 2b shows part of another embodiment of the cleaning apparatus, wherein two optical fibres 12, 120 are connected at opposite ends to the same cavity 14. Preferably both fibres have a concave output surface, shaped to focus the ultrasound waves inside cavity 14. The concavities of the output surfaces of the fibres are preferably chosen to create an off-axis local maximum in the resonant mode shape, e.g. close to the hole 24.

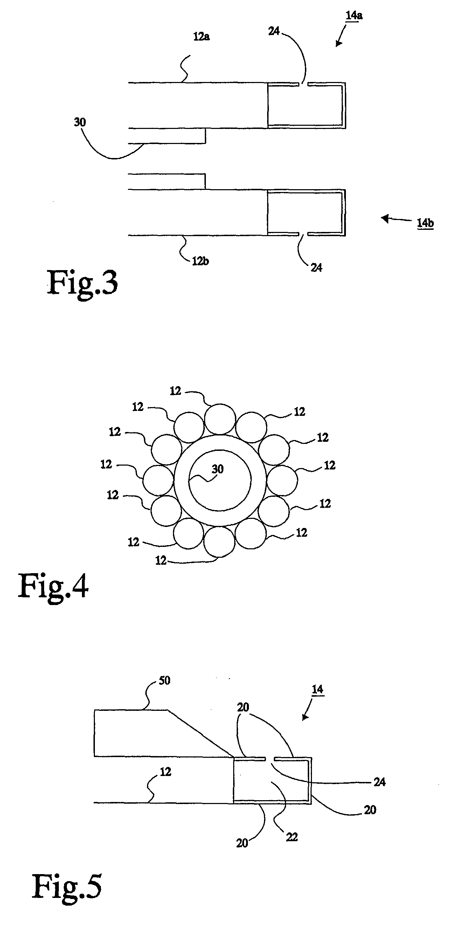

In operation, when the embodiment of figure 3b is used, the combination of fibres 12, 120 is moved through tube 16, one fibre 12 extending to one end of tube 16 and beyond, the other fibre 120 extending to the other end of tube 16 and beyond. Modulated light is supplied through both fibres 12, 120 from opposite ends of tube 16 respectively. In this way fibres 12, 120 both supply energy to cavity 14, so that the pressure amplitude of the ultrasound waves is higher than in the embodiment in which one fibre is used. Preferably, the respective modulation phases of the light that is applied to the respective fibres are phase locked to each other, so that a maximum sound amplitude is realized.

Figure 3 shows part of another embodiment of the cleaning apparatus, wherein in a central supply tube 30 is provided, with a bundle of a plurality of optical fibres 12a,b attached to the central supply tube. Respective cavities 14a,b are attached to the tips of the optical fibres 12a,b. Two optical fibres 12a,b with corresponding cavities 14a,b are shown in the bundle by way of example, but a greater number may be used. Coupling holes 24 are provided in the respective cavities preferably facing outwardly from the bundle and not inward of the bundle.

Figure 4 shows another embodiment of the bundle in cross-section, wherein the optical fibres 12a,b of the bundle are arranged successively along a circle around the supply tube 30. In this case coupling holes 24 (not shown) are preferably provided in the respective cavities facing radially outward. It should be appreciated, that a central location is preferred for supply tube 30, with the fibres 12 located along the perimeter of supply tube 30, to ensure radiation of sound equally in all directions, but other locations may be used, providing the bundle of fibres 12 on one side of the supply tube 30 for example.

In operation, liquid is supplied through supply tube 30 and pulsed laser light is supplied through all of optical fibres 12 to excite resonant ultrasound standing waves in the cavities 14 at the tips of the optical fibres 12. Supply tube 30 supplies liquid. This has the advantage that liquid can be supplied without obstruction to the location where cavities 14 transmit sound waves to the interior surface of tube 16. Alternatively, liquid may be drained through tube 30, which then acts as drain tube instead of supply tube.

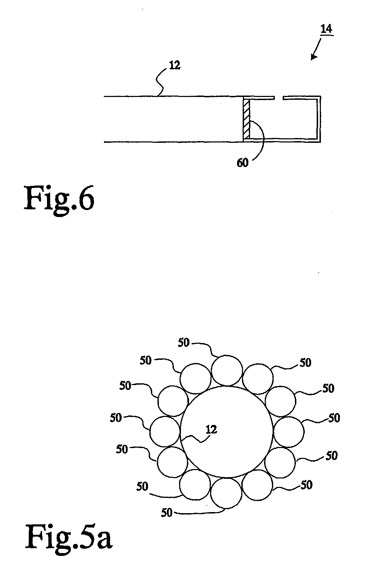

As an alternative the supply tube may be arranged for example around a fibre 12, or a plurality of fibres, the supply tube having an inner diameter that leaves room to supply liquid along the fibre 12 or fibres. In this alternative, spacer structures may be provided so that the interior surface of the supply tube is held at a fixed distance from the fibre 12 or fibres. LTV-fibres may serve as longitudinal spacers, see figures 5 and 5a.

Optical fibre 12 or optical fibres 12 are preferably made of a biocompatible material such as germanium oxide.

Preferably a motor driven pulling arrangement is provided to pull the tip of optical fibre 12 (or the bundle of optical fibres) through tube 16 at a substantially constant speed. This ensures equal cleaning. Alternatively a motor driven arrangement may be used to push the optical fibre 12 through tube 16. As further alternative, if tube 16 allows this, pressure applied to tube 16 may be used to push the tip of optical fibre 12 through tube, or a vacuum may be used to pull the tip. In a further embodiment the speed of the pulling motor may be slowed down locally to achieve increased cleaning at locations of the interior surface of tube 16, where this is known to be required. Alternatively, a stepped movement of the tip of optical fibre may be used.

A continuous series of light pulses is preferably applied through optical fibre 12 while the optical fibre 12 is moved through tube 16. Alternatively, bursts of pulses may be used, for example each time when the tip has been stepped to a next position, or with a burst frequency selected so that between successive bursts the tip moves less than the size of a spot where the peak of the sound amplitude is achieved on the interior surface of tube 16.

Achieving a sufficient amplitude of the emitted sound waves is more important than achieving sound waves over a large area. Therefore, holes 24 are preferably kept small in size and number to facilitate a high-pressure amplitude to build up of the standing waves within cavity 14. Cleaning of a large area is achieved by moving the holes 24 relative to the interior surface of tube 16.

Instead of holes 24, valves may be provided in the walls 20 of the cavity or cavities. These valves may be arranged to open only once more than a threshold amount of over-pressure due to the standing sound waves has developed in cavity 14, relative to the surrounding liquid. In this way pressure amplitude builds up in the cavity 14, without loss of resonant energy through the valve until sufficient amplitude has been reached. In this way a higher amplitude can be realized in the sound waves that are emitted from the cavity 14 towards the interior surface of tube 16.

Valves of this type may be realized for example by using a resilient material for wall 20, at least an on the sides of a hole 24, so that the resilience acts to press wall materials from opposite sides of a hole against each other, unless more than a threshold pressure amplitude has built up in the standing waves inside the cavity 14, which pushes the wall materials around the hole 24 aside. Of course, other types of valves that have the same effect of opening only during pressure peaks may be used instead. Instead of holes or valves, a membrane may be provided that transmits pressure variations from the inside of cavity 14 to the liquid outside the cavity.

Figure 5 shows a further embodiment wherein a further optical fibre 50 is provided adjacent to optical fibre 12. The end of further optical fibre 50 is constructed so that light from further optical fibre 50 is coupled from further optical fibre 50 towards the interior surface of tube 16 where, or near where, sound emitted from cavity 14 has maximum amplitude on the interior surface. A UV (Ultraviolet) source (not shown) is coupled to the terminal of further optical fibre 50 outside tube 16. Further optical fibre 50 is of a material that transmits UV light.

In operation, during cleaning, UV radiation is supplied through further optical fibre 50, and emitted towards the interior surface of tube 16 to perform UV disinfection immediately after detachment of material from the interior surface by means of sound pressure. Thus the interior surface can be additionally cleaned shortly after pollution has been removed by ultrasound pressure variation and before other pollution can attach itself to the surface. Without a well-cleaned surface, UV disinfection would be much less effective. Preferably, an ozone-forming wavelength between 100 and 190 nanometre is present in the applied UV-spectrum. This will produce free oxygen radicals, which are effective against pyrogens and prions. Also longer UV wavelengths are germicidal and could be applied, e.g. 254 nanometre. The UV radiation may be used in combination with chemicals that are added to the supplied liquid.

In another embodiment the UV radiation may be supplied through the optical fibre 12 that also supplies the light pulses to excite the standing sound waves in cavity 14. In this case the UV radiation is coupled from the optical fibre 12 to the interior surface of tube 16.

Figure 5a shows another embodiment of the bundle in cross-section, wherein multiple further optical fibres 50 of the bundle are arranged successively along a circle around optical fibre 12. In this embodiment multiple coupling holes 24 (not shown) are provided in the sidewalls of the cavity that is attached to fibre 12. The UV fibres 50 are shaped to direct UV radiation towards respective locations where respective ones of the holes 24 direct ultrasound to tube 16. In operation UV radiation is supplied through multiple further optical fibres 50, so that UV light is radiated towards the interior surface of tube 16. In this way, there is no need to rotate the bundle of fibres to clean the entire surface.

It should be appreciated that other configurations are possible, such as the one shown in figure 4, with a central tube 30 for liquid supply, using around the perimeter of the central tube 30 alternately fibres for supplying modulated light to excite ultrasound and fibres for supplying UV radiation directed towards locations where the holes from another fibre direct ultrasound to tube 16. In this case, during operation modulated light and UV light are applied to alternate fibres. In another configuration, further fibres (not shown) for UV radiation may in turn be attached to respective fibres 12 that are provided along the perimeter of central tube 30. In each of these embodiments a plurality of fibres for supplying UV radiation is provided, each in conjunction with a respective hole 24 in a cavity.

Figure 6 shows another embodiment, wherein a light-absorbing layer 60 has been added at the tip of optical fibre 12. In this embodiment the liquid in cavity 14 is not directly heated by light from optical fibre 12, but indirectly through heating of absorbing layer by means of pulsed light. This has the advantage that the light need not have a wavelength that is absorbed by the liquid in cavity 14. When the absorbing material is selected according to the wavelength, any wavelength may be used, for example a wavelength for which a suitable laser source 10 is available or which is minimally absorbed in optical fibre 12. A disadvantage compared to direct heating is that the heating of water is slower, so that more energy has to be supplied to reach standing waves with the same amplitude as in the case of direct heating.

Light absorbing layer 60 may be provided elsewhere, for example at the end wall of cavity 14 that is opposite to the tip of optical fibre 12. Instead of heating by means of laser light other means of exciting standing sound waves in cavity 14 may be used. For example, a piezo-electrical transducer may be provided in cavity 14, and electrical conductors may be connected to this transducer from outside tube 16 to excite the standing waves. Although this is a suitable alternative to excitation by means of laser light, laser light is more effective in exciting high amplitudes in the standing wave pattern. This is due to the liquid to vapour transformation that creates large pressure waves.

## Claims

1. A method of cleaning an inner surface of an elongated narrow interior space of an object such as the lumen of an endoscope or catheter, the method comprising
- supplying a liquid to the interior space;
- moving a liquid-filled, sound-resonant cavity through the interior space, attached to an energy supply channel;
- feeding excitation energy through the energy supply channel, the excitation energy having a power level that is modulated with a frequency that substantially corresponds to a sound-resonance frequency of the cavity;
- converting the excitation energy in the cavity to pressure variations in the liquid in the cavity, so that a standing sound wave is excited in the cavity;
- feeding sound pressure variations from the cavity to the inner surface of the interior space.

2. A method according to Claim 1, wherein the energy supply channel comprises an optical fibre, the excitation energy being fed to the cavity by applying amplitude modulated light through the optical fibre with a modulation frequency at said frequency.

3. A method according to Claim 2, wherein the light is fed to the liquid in the cavity and the light has a wavelength in an absorption band of the liquid.

4. A method according to Claim 3, the cavity comprises material that is absorptive for the light, the material heating the liquid to excite the standing wave.

5. A method according to any one of the preceding Claims, comprising supplying UV light locally to a location on the inner surface when, or shortly after the sound pressure variations are supplied to the location from the cavity.

6. A method according to any one of the preceding Claims, wherein the liquid is water or a water-based solution.

7. A method according to any one of the preceding Claims, comprising performing a process step wherein the object is moved into a medium that contains biological material, and performing the steps of supplying the liquid and moving the energy supply channel attached to the cavity through the interior space after said process step.

8. A cleaning apparatus for cleaning an inner surface of an elongated narrow interior space of an object such as the lumen of an endoscope or catheter, the apparatus comprising
- an elongated energy supply channel, fit to be moved through the interior space, the supply terminal having a terminal for connection of a energy source outside of the interior space;
- a sound-resonant cavity coupled to energy supply channel to excite an standing sound wave in the cavity by means of conversion of energy that is supplied from the energy supply channel, the cavity being fit to be moved with the energy supply channel through the interior space; the cavity having a pressure transmission interface for transmitting pressure amplitude variations of the standing waves from inside the cavity to the interior space outside the cavity.

9. A cleaning apparatus according to Claim 8, wherein the cavity is filled with liquid.

10. A cleaning apparatus according to Claim 8 or 9, comprising an energy source coupled to the terminal and arranged to feed excitation energy through the energy supply channel, the energy source being arranged to modulate the power level of the excitation energy at a frequency that substantially corresponds to a sound-resonance frequency of the cavity.

11. A cleaning apparatus according to Claim 10, wherein the energy supply channel comprises an optical fibre, the energy source being a source of amplitude modulated light modulated at said frequency.

12. A cleaning apparatus according to Claim 11, wherein the cavity comprises material that has an absorption band at a wavelength of the light.

13. A cleaning apparatus according to Claim 12, wherein the cavity is filled with liquid that has an absorption band at the wavelength of the light.

14. A cleaning apparatus according to Claim 12, wherein the cavity is filled with liquid in contact with said material.

15. A cleaning apparatus according to any one of Claims 8 to 14, comprising a bundle of optical fibres, each coupled to a respective cavity to excited standing sound waves in the cavities by means of modulated light.

16. A cleaning apparatus according to any one of Claims 8 to 15, wherein the pressure transmission interface comprises a hole in a wall of the cavity.

17. A cleaning apparatus according to any one of Claims 8 to 16, wherein the pressure transmission interface comprises a valve in a wall of the cavity, the valve being arranged to open when a pressure of the standing wave exceeds a threshold value.

18. A cleaning apparatus according to any one of Claims 8 to 17, wherein an output surface of the fibre that interfaces with the liquid in the cavity is shaped to focus sound waves, so that in operation an on-axis or off-axis local amplitude maximum of the sound waves is formed.

19. A cleaning apparatus according to any one of Claims 8 to 18, comprising a liquid supply or drainage tube that runs parallel to the energy supply channel and is fit to be moved through the interior space with the energy supply channel and the cavity.

20. A cleaning apparatus according to any one of Claims 8 to 19, comprising a UV light source coupled to the energy supply channel, the energy supply channel being arranged to pass UV light from the UV light source to the inner surface substantially to a location where the sound pressure variations reach the inner surface from the cavity.

21. A cleaning apparatus according to Claim 20, wherein the energy supply channel comprises a first optical fibre that terminates in the cavity, for supplying light of a first wavelength to the cavity to excite the standing wave, and a second optical fibre, arranged to couple UV light from the second optical fibre to said location.

22. A cleaning apparatus according to Claims 20 and 21, comprising a bundle of second optical fibres, each coupled to a UV light source, and each arranged to couple UV light from the second optical fibre to said location.
